(19) Europäisches Patentamt
European Patent Office
Office européen des brevets

(11) EP 3 393 455 B1

(12) EUROPEAN PATENT SPECIFICATION

(45) Date of publication and mention of the grant of the patent:
**21.07.2021 Bulletin 2021/29**

(21) Application number: **16819318.3**

(22) Date of filing: **21.12.2016**

(51) Int Cl.:
*A61K 9/10* (2006.01) *A61K 47/12* (2006.01)
*A61K 47/14* (2017.01) *A61K 31/365* (2006.01)
*A61K 31/429* (2006.01) *A61P 33/10* (2006.01)

(86) International application number:
**PCT/EP2016/082188**

(87) International publication number:
**WO 2017/108954 (29.06.2017 Gazette 2017/26)**

(54) **INJECTABLE FORMULATION OF A MACROCYCLIC LACTONE AND LEVAMISOLE**

INJIZIERBARE FORMULIERUNG AUS EINEM MAKROCYCLISCHEN LACTON UND LEVAMISOL

FORMULATION INJECTABLE À BASE D'UNE LACTONE MACROCYCLIQUE ET DE LÉVAMISOLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2015 US 201562270721 P**

(43) Date of publication of application:
**31.10.2018 Bulletin 2018/44**

(73) Proprietor: **Intervet International B.V.**
**5831 AN Boxmeer (NL)**

(72) Inventors:
- **VETTORATO, Luis, Fernando**
  **04794-000 Sao Paulo SP (BR)**
- **ASSIS, Ulisses, Rafael, Santos**
  **12730-340 Cruzeiro SP (BR)**
- **CORTEZ, Luiz, Daniel**
  **12730-340 Cruzeiro SP (BR)**

(74) Representative: **Intervet International B.V.**
**Hertford Road**
**Hoddesdon, Hertfordshire EN11 9BU (GB)**

(56) References cited:
**WO-A1-2013/043064 WO-A2-2008/072985**
**WO-A2-2013/030702**

- **DATABASE WPI Section Ch, Week 201628 Thomson Scientific, London, GB; Class A96, AN 2015-736022 XP002767978, FANG Y; SUN H; ZHONG R; ZHOU D: "Anthelmintic nano-suspension formulation for sheep and goats, comprises preset amount of water and nano-suspension formulation stock solution comprising nano suspended particles, stabilizer and dimethylsulfoxide", -& CN 104 983 680 A (CHINESE ACAD SCI NORTHEAST INST GEOGRAPH) 21 October 2015 (2015-10-21)**
- **DATABASE WPI Section Ch, Week 200321 Thomson Scientific, London, GB; Class B02, AN 2003-211551 XP002767979, DAI X; PAN Z; WANG Y: "Veterinary mixed injection useful in dispelling and killing nematode in pigs, cattle and sheep and also simultaneously dispelling external parasite, contains levamisole or its salts", -& CN 1 375 291 A (WANG Y) 23 October 2002 (2002-10-23)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 393 455 B1

## Description

### FIELD OF INVENTION

This invention relates to injectable formulations with enhanced stability for combating parasites in and on animals, comprising a macrocyclic lactone and levamisole and methods for eradicating, controlling, and preventing parasitic infections and infestations in or on an animal comprising administering the compositions of the invention to the animal in need thereof.

Endoparasites and ectoparasites commonly cause clinical disease in livestock animals and have significant adverse economic effects on farming economies when present at subclinical levels.

The most frequently encountered endoparasites are the group of worms referred to as nematodes. The nematodes are found in the intestinal tract, heart, lungs, blood vessels and other body tissues of animals and are a primary cause of anemia, weight loss and malnutrition in the infected animals. The nematodes most commonly found to be the infecting agents of ruminants include *Haemonchus* and *Ostertagia* generally found in abomasum; *Cooperia, Trichostrongylus* and *Nematodirus* generally found in the intestinal tract, and *Dictyocaulus* found in the lungs.

Ectoparasites are parasites that live on the outside of their host rather than within the host's body. The common ectoparasites in livestock animals are ticks (*R. microplus*), horn flies (*Haematobia irritans*), grubs (*Dermatobia hominis*) and screwworms (*Cochliomyia hominivorax*).

Treatment of animals to prevent infestation by any of the above-mentioned parasites, or to reduce or control the proliferation of these parasites in animals is thus important.

Meanwhile the problem has arisen that some parasites develop a resistance to antiparasitic drugs like ivermectin. The resistance occurs when a strain of a parasite is able to tolerate doses of an active ingredient that is efficacious against other populations of parasites of the same species. This characteristic is inheritable.

After the use of macrocyclic lactones (ML) for almost two decades in cattle in Brazil, several reports on resistant endoparasites and ectoparasites in sheep, cattle and goats were published.

The discovery of novel anti-parasitics with equal or better qualities than macrocyclic lactones seems to be a distant reality in the veterinary pharmaceutical industry. Therefore, the chemical classes available nowadays must be used in a rational way, with a view to achieving high percentages of efficacy against endoparasites and ectoparasites especially in ruminants and delaying the occurrence of resistant strains. Therefore a stable formulation for a combination of a ML and levamisole would be desirable. However, such combinations have been difficult to formulate due to the distinct chemical characteristics of each of these active ingredients.

Accordingly, there is a need for a stable formulation capable of including ML compounds together with levamisole. One of the approaches to overcome these distinct chemical characteristics is to keep the ML compound and levamisole separated in the formula, with one of the active ingredients suspended and the other compound in solution.

WO 00/74489 represents a recent attempt to formulate a combination avermectin/milbemycin and levamisole product. In this formulation, one active ingredient is dissolved in an organic liquid phase and the other active ingredient is dissolved in an aqueous liquid phase. Ashmont NZ 280085/280134 discloses injectable ML anthelmintic formulations (eg; abamectin or ivermectin) where an alcohol (such as benzyl alcohol-long since used as a preservative in injectable formulations) acts as a co-solvent with a vegetable oil vehicle (eg; soyabean oil, sesame oil or corn oil).

Associations of avermectines and levamisole have also been described before, in patent application WO 00/061068 and in patent application WO 2004/009080. BR P19802431 discloses a combination of ivermectin and levamisole in an oily formulation where the ivermectin is in particles. WO2011/161209 discloses an injectable formulation of a macrocyclic lactone and levamisole.

This application does not disclose the use of triacetin in the formulation. The formulation comprises dimethylacetamide, castor oil and medium chain triglyceride oil such as caprilic/capric triglyceride. CN1375291 discloses a veterinary injection formulation containing levamisole and ivermectin in a carrier vehicle that is not triacetin. This formulation also comprises a dispersing agent (e.g. dimethylacetamide, polyethylene glycol, glycerol formal), a suspending agent (e.g. aluminium stearate) and a surfactant (e.g. esters synthesized from glycerol (triacetin)).

EP 0413538 discloses an injectable formulation of ivermectin and triacetin. NZ594610 and WO 2013/030702 A2 disclose an anthelmintic formulation comprising levamisole, ivermectin and N-methyl-2-pyrrolidone. The documents also refer to one or more solvents chosen from the group comprising N-methyl-pyrrolidone, N-pyrrolidone, glycerol formal and triacetin to dissolve the formulations disclosed. WO2008/072985 discloses a pour on formulation containing a macrocyclic lactone, levamisole and triacetin.

The combination of a macrocyclic lactone with levamisole presents a number of challenges from a formulation solubility and stability perspective, as levamisole is known to be water soluble acid-stable, while the macrocyclic lactones are not water soluble and are acid labile and are subject to hydrolytic degradation.

The present invention describes a composition of a macrocyclic lactone and levamisole that is an efficacious injectable veterinary dosage form involving only few excipients, but have good re-suspendability, stability, and shelf life,

thereby allowing for increased antiparasitic efficacy after administration to animals in need thereof and reduced resistance in the parasites.

SUMMARY OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method of treatment of the animal body by therapy.
An embodiment of the invention is an injectable pharmaceutical formulation comprising a macrocyclic lactone selected from the group comprising abamectin, doramectin, eprinomectin, ivermectin and moxidectin and levamisole, and triacetin as the sole liquid carrier and a dispersion stabilizer selected from metallic stearates of calcium, zinc, magnesium or aluminum, wherein the macrocyclic lactone is in solution and the levamisole is in suspension and has an average effective particle size $D_{50}$ between 100 - 150 $\mu$m when measured with a Beckman Coulter LS13320 Particle size analyzer.
In a preferred embodiment, the formulation comprises aluminum stearate. In another embodiment, the formulation further comprises a preservative, preferably butylated hydroxytoluene (BHT). In another embodiment, the formulation has a viscosity in the range of 10 to 100 mPas (10 to 100 cP), preferably 20 to 50 mPas (20 to 50 cP).
In another embodiment, the levamisole is the hydrochloride salt.
In another embodiment, the macrocyclic lactone is ivermectin and is present in about 1 or about 4% w/v.
In another embodiment, the macrocyclic lactone is moxidectin and is present in about 1 or about 4% w/v.
In another embodiment, the macrocyclic lactone is eprinomectin and is present in about 1 or about 4% w/v.
In another embodiment, the formulation comprises:

- about 0.5- 10% w/v ivermectin,
- about 15- 20% w/v levamisole,
- about 0.1-1% w/v aluminium monostearate,
- about 0.1-1% w/v butylated hydroxytoluene (BHT) and
- triacetin, qs 100% (v/v).

Another embodiment of the invention is a formulation as described above for use in treating or preventing infestation or infection of internal parasites and external parasites in animals.
An alternative embodiment of the invention is a process for preparing the claimed injectable pharmaceutical formulation comprising

a) dissolving a macrocyclic lactone in triacetin;
b) mixing the macrocyclic lactone solution with levamisole; and
c) milling the mixture of step b) to achieve an effective average particle size $D_{50}$ of between 100 - 150 $\mu$m when measured with a Beckman Coulter LS13320 Particle size analyzer,

wherein in step b) aluminum stearate is added to the solution of step a) before or after adding the levamisole.
In another embodiment, the temperature of step c) is kept below 50°C.

DETAILED DESCRIPTION

We have now discovered formulations of a combination of one or more macrocyclic lactone, especially ivermectin, moxidectin, eprinomectin or doramectin, with levamisole, in a triacetin carrier which have long duration of action in vivo, and which have beneficial properties e.g. injection site tolerability, stability on storage, acceptable viscosity, syringeability across a wide temperature range, and good bioavailability.
It has been found that especially beneficial formulations have an average effective particle size $D_{50}$ of between 100 - 150 $\mu$m.
The formulations of the invention are useful in the treatment of economically important parasite infections, including endo-parasites such as gastrointestinal roundworms. The formulations are also useful in the treatment of ectoparasites (arthropods) such as ticks, mites, lice, blowfly, biting insects, migrating dipterous larvae, etc.
A macrocyclic lactone (ML) compound in solution with levamisole in a particulate form (suspended) in triacetin as liquid carrier is advantageous as it provides stable formulations for ML compounds (e.g. ivermectin, moxidectin and eprinomectin) in combination with levamisole.
The ML compounds are potent endo and ectoparasitic agents. The compounds which belong to this class are

either natural products or are semi-synthetic derivatives thereof. The structure of two classes of ML compounds, avermectins and milbemycins, are closely related and they both share a complex 1,6-membered macrocyclic lactone ring; the avermectins comprise a disaccharide substituent in the 1,3-position of the lactone ring, which the milbemycins do not. The macrocyclic lactones are products, or chemical derivatives thereof, of soil microorganisms belonging to the genus *Streptomyces* (see The Merck Veterinary Manual 8th Ed., 1998, pages 1812-1814).

The ML e.g. avermectins, milbemycins and derivatives thereof are selected from the group which includes but is not limited to, abamectin, doramectin, emamectin, eprinomectin, ivermectin, and selamectin (avermectin and derivatives thereof), milbemycin D, milbemycin oxime, lepimectin, and moxidectin (milbemycin and derivatives thereof) and mixtures thereof.

One particularly contemplated ML parasiticide is ivermectin. Ivermectin is a semi-synthetic derivative of avermectin, and is generally produced as a mixture of at least 80% 22,23-dihydroavermectin $B1_a$ and less than 20% 22,23-dihydroavermectin $B1_b$. Ivermectin is disclosed in US Patent 4,199,569. Ivermectin has been used as an antiparasitic agent to treat various parasitic diseases since the mid-1980's.

Other ML parasiticides include, for example the following:

Abamectin This compound is, for example, identified as avermectin $B1_a/B1_b$ in U.S. Patent 4,310,519. Abamectin contains at least 80% of avermectin $B1_a$, and not more than 20% of avermectin $B1_b$.

Doramectin This compound is known as 25-cyclohexyl-avermectin $B_1$. Its structure and preparation are discussed in, for example, US Patent 5,089,480.

Emamectin This compound also is known as 4"-deoxy-4"-epi-methylaminoavermectin $B_1$. Its preparation is discussed in, for example, US Patent Nos. 5,288,710 and 5,399,717.

Eprinomectin This compound is known as 4"-epi-acetylamino-4"-deoxy-avermectin $B_1$. It was developed for use in all cattle classes and age groups.

Selamectin This compound also is known as 25-cyclohexyl-25-de (1-methylpropyl)-5-deoxy-22, 23-dihydro-5-(hydroxyimino)-avermectin B1 monosaccharide.

Milbemycin and milbemycin oxime This compound also is known as B41. It is isolated from the fermentation broth of a Milbemycin-producing strain of *Streptomyces*. The microorganism, fermentation conditions, and isolation procedures are discussed in, for example, US Patents 3,950,360 and 3,984,564.

Moxidectin This compound is discussed in, for example, US Patent. 4,916,154.

Lepimectin This is a chemically modified milbemycin macrolide (6*R*,13*R*,25*R*)-5-*O*-demethyl-28-deoxy-6,28-epoxy-13-[(Z)-[(methoxyimino)phenylacetyl] oxy]-25-methylmilbemycin B mixture with (6*R*,13*R*,25*R*)-5-*O*-demethyl-28-deoxy-6,28-epoxy-25-ethyl-13-[(Z)-[(methoxyimino) phenylacetyl] oxy] milbemycin B.

Preferred ML compounds are ivermectin, moxidectin and eprinomectin.

The ML compounds are present in the range of between 0.01-10% w/v, 0.5-10%w/v, 0.5-8% w/v, 1-6% w/v, 1.5 - 5% w/v, or 1.6%- 4% w/v, or are present at about 1% w/v, or about 4%w/v.

Levamisole (IUPAC name (S)-6-Phenyl-2,3,5,6-tetrahydroimidazo[2,1-b][1,3]thiazole) as used in this specification includes levamisole base, levamisole hydrochloride, levamisole phosphate together with other salts and forms. In one embodiment preferably levamisole hydrochloride is used. It has been found that formulations of levamisole hydrochloride were especially stable.

Levamisole is present in the range of between about 0.1-40% w/v, about 10-35% w/v, about 12-30% w/v, about 15-25% w/v, or about 15-20 % w/v, or is present at about 18% w/v, or about 18.8% w/v.

Good results were obtained with a formulation comprising about 1.0 - 4.0% w/v ivermectin and about 15 - 20% w/v levamisole hydrochloride. Good results were also obtained with a formulation comprising about 1.0 % w/v ivermectin and about 18.8 % w/v levamisole hydrochloride. Good results were also obtained with a formulation comprising about 4.0% w/v ivermectin and about 18.8 % w/v levamisole hydrochloride.

The complicated nature of prior art formulations is due in part to the different formulation requirements of the actives. Ivermectin, moxidectin and eprinomectin are substantially insoluble in water, whereas levamisole is water soluble.

The formulation according to the invention comprises the Macrocyclic Lactone in solution and the levamisole in suspension. A solution is a (liquid) mixture of two or more components that form a single phase that is homogeneous down to the molecular level. A suspension consists of insoluble solid particles dispersed in a liquid medium, with the

solid particles accounting for about 0.1% to about 30% w/v of the suspension.

In the formulation according to the invention triacetin is the sole liquid medium (carrier).

Triacetin is the triacetic acid ester of glycerin, also referred to as glyceryl triacetate which has the following formula:

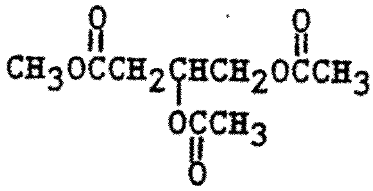

The amount of triacetin in the formulation is between about 20% v/v and 80 % v/v.

The formulation according to the invention exhibits desirable properties which are useful characteristics for the administration of relatively high concentrations of levamisole and/ or of a macrocyclic lactone compound such as ivermectin and maintains a low viscosity even at high drug (macrocyclic lactone and/or levamisole) loads in the formulation.

The relatively high levels of ivermectin and levamisole in these formulations enable administration of low injection volumes of formulation, without loss of the beneficial properties mentioned above.

The formulations according to the invention allow for a low dose volume, as low as 1 ml per 50 kg of body weight. Such low injection volume allows the use of such formulations in (cattle) animals of a broad weight-band, including heavy animals. An example would be that for an animal of 600 kg bodyweight an injection volume/ animal of 12 ml would be required. Therefore the formulation is especially suited for cattle animals with a body weight of more than 450 kg.

The formulations are physically and chemically stable. In addition, as the formulation excludes water, the issue of incompatible pH requirements is alleviated, enabling the two actives to stably co-exist in different phases.

The formulations of a ML and levamisole of the present invention must be stable to be of commercial use. In this specification, a commercially acceptable endectocide/ectocide formulation is one which is stable at room temperature (30°C ± 2°C / 65% RH ± 5%, according to zone IV conditions) for a period of at least 24 months. In conditions of accelerated testing, at 40°C ± 2°C / 75% RH ± 5%, or even at 50°C ± 2°C / 90% RH ± 5% this requires the potency of the actives within the formulation to remain within specified and acceptable limits for 6 months. Stability is calculated by the following formula:

$$\frac{(I - F)*100\%}{\quad} = \%\text{ of degradation}$$

wherein I is the initial concentration of the active ingredient at the beginning of the testing period and F is the final concentration of the active ingredient at the end of the testing period. The degradation of the active ingredients should be less than 5% over the 6 month testing period. As it can be seen in Table 4 and 5, formulations of the current invention are stable, even under accelerated conditions.

The formulations of the present invention must also be of acceptable viscosity to be administered by syringe, preferably a syringe with a 40 x 1.2 mm needle.

Viscosity is a measure of a fluid's resistance to flow. It describes the internal friction of a moving fluid. The centipoise (cP) is a non-SI (non-System International) measurement unit of dynamic viscosity in the centimeter gram second (CGS) system of units. (See The Condensed Chemical Dictionary, 5th Ed., 1956, page 1159).

In an embodiment of the invention, the viscosity of the formulation is between about 1 to 150 mPas, about 10 to 100 mPas or about 20 to 50 mPas (about 1 to 150 cP, about 10 to 100 cP or about 20 to 50 cP).

Syringeability is the ability of a product to be successfully administered by a syringe and an appropriate needle. By syringeable it is meant that the suspension can be withdrawn easily from an ampoule/ vial into a syringe with a conventionally used needle for veterinary applications to (bovine) animals and subsequently injected from such a syringe through the needle subcutaneously (sc) to the (bovine) animal. This is especially important in case the temperature of the environment is low.

Syringeability testing was conducted at room temperature by application of a constant force on the piston of a 5 ml syringe with a needle (needle 40 x 1.2 mm or needle 40 x 1.6 mm). A successful test required no clogging while expelling the entire contents of the syringe. As it is shown in Table 4 the formulations of the current invention have an improved syringeability.

The effective average particle size of the levamisole is also important for the stability and syringeability of the formulation, especially in combination with the viscosity.

By "particle" it is meant mobile, un-dissolved, solid matter suspended in a liquid. Average effective particle size

is adequately measured by laser diffraction. Average effective particle size (particle size) is expressed in units of microns. $D_{50}$ is the volume (v) median diameter sometimes shown as D[v,0.5] or D0.5. It is the value of the particle diameter at 50% in the cumulative distribution. It is an important parameter characterizing particle size. For example, if $D_{50}$ = 50 μm, then 50% of the particles in the sample are larger than 50 μm, and 50% smaller than 50 μm. $D_{50}$ is usually used to represent the particle size of a group of particles. Particle size is measured with a Beckman Coulter LS 13320 Particle Size Analyzer. Particularly, the present inventors have found that advantageous effects are exerted when the levamisole has a $D_{50}$ of between 100 - 150 μm.

The present inventors conducted extensive studies and consequently have found that the formulations according to the invention have a good physical stability - dispersibility, and a low viscosity, a good syringeability even under different temperature conditions and a good (long term) storage stability.

It has been also found that the inclusion of a dispersion stabilizer (further) improves the physical stability of the formulation.

This means in the formulations of the current invention only a limited sedimentation of the particles over time is observed even though the viscosity of the composition is low. This is surprising because generally suspension formulations with a higher viscosity are known to have a better physical stability and dispersibility.

Dispersion stabilizers help active pharmaceutical ingredients stay suspended in the formulation, therefore improve dispersibility and prevent caking at the bottom of the container/ flask. "Caking" is the process in which almost all particles in the sediment become interconnected, making it impossible to re-suspend. One of the properties of a well-formulated suspension is that it can be easily re-suspended by the use of moderate agitation or shaking. This has been achieved with the formulation according to the invention that is easy to resuspend, especially in bottles or vials that contain 50ml or 500 ml of the formulation.

According to the invention, the dispersion stabilizer is a metallic stearate of calcium, zinc, magnesium or aluminum.

The aluminum stearates are particularly effective and generally mono-, di- or tribasic stearates can be used.

In one embodiment aluminum monostearate is used. In an alternative embodiment aluminum distearate is used.

In a specific embodiment the use of the dispersion stabilizer does not require the presence of other surfactants in the formulation.

The dispersion stabilizer (e.g. aluminum stearate) is present in the formulation between about 0.1% w/v and 5 % w/v or about 0.1% w/w to 2% w/v or 0.5% w/v.

By "w/v" is meant weight/volume, i.e. "1% w/v" means 1 g in 100 ml of the formulation. "v/v" means volume per volume, and 1% v/v means 1 ml, in a total of 100 ml.

The formulation according to the current invention may further comprise additional pharmaceutical excipients known in the art. Such pharmaceutical excipients are e.g. described in "Gennaro, Remington: The Science and Practice of Pharmacy", (20th Edition, 2000).

Such ingredients include preservatives, chelating agents, antioxidants and stabilizers.

Suitable minerals and vitamins that can be included in the veterinary formulation are known.

In addition to the triacetin the formulation can contain an antioxidant such as propyl gallate, BHA (butylated hydroxy anisole), BHT (butylated hydroxy toluene), monothioglycerol and the like. The antioxidants are generally added to the formulation at rates of from 0.01 to 2.0% (w/v).Exemplary preservatives include methyl p-hydroxybenzoate (methylparaben) and propyl p-hydroxybenzoate (propylparaben). Exemplary chelating agents include edetate sodium. Exemplary antioxidants include butylated hydroxyanisole and sodium monothioglycerol.

Preferred formulations of the present invention comprise:

- **Component 1**-Macrocyclic Lactone compound as claimed
- **Component 2-** Levamisole
- **Component 3-** Triacetin
- **Component 4-** dispersion stabilizer as claimed, e.g. aluminumstearate

Additional components can be added to this basic formulation.

- **Additional component 1-** Additional active(s) of which at least one is preferably an anthelmintic active. These can be either dissolved or suspended if insoluble, or both.

- **Additional component 2-** Minerals/Vitamins

- **Additional component 3-** Preservatives.

Preferred components and their concentration ranges and examples are set out in Table 1.

**Table 1:**

| | Function | Examples | % W/V |
|---|---|---|---|
| Component 1 | Macrocyclic lactone | ivermectin, moxidectin or eprinomectin | 0.1-10% |
| | | | |
| Component 2 | Levamisole | levamisole hydrochloride | 0.1 - 40% |
| Component 3 | organic solvent | Triacetin | 20-80% |
| Component 4 | Dispersion stabilizer | Aluminium stearate | 0.1 - 1% |
| Additional Component 1 | Additional anthelmintic | | 0-25% |
| Additional component 2 | Minerals | Mineral salts or chelates | 0-5% |
| Additional component 3 | Preservatives | Benzoic Acid, Potassium Sorbate and Parabens, Benzyl Alcohol, methylhydroxytoluen, butylated hydroxytoluene (BHT) | 0-1% |

In an embodiment of the invention, the levamisole concentration is between about 0.1 to 40% w/v, about 7 to 30 % w/v, about 10 to 25 % w/v, about 15 to 20 % w/v or about 17 to 19 % w/v or about 18% w/v.

In an embodiment of the invention, the ivermectin concentration is between about 0.1 to 10% w/v, about 0.4 to 10% w/v, about 0.5 to 10% w/v, about 1 to 8% w/v, or about 2 to 4 % w/v, or about 1% or about 4%.

In the present invention, the particle size $D_{50}$ of the levamisole is between 100 - 150 μm, or about 110μm to about 140μm.

## Methods of producing the formulations according to the invention

In another aspect of the invention there is provided a method of preparing the formulation according to the invention. This manufacturing process is easy and has only a few steps. The desired particle size of the suspension is obtained by milling the suspension. This is beneficial because the micronisation of the levamisole before addition to the formulation results in stability problems.

The current invention provides a process for preparing an injectable pharmaceutical formulation as claimed comprising

a) dissolving a macrocyclic lactone in triacetin;
b) mixing the macrocyclic lactone solution with levamisole; and
c) milling the mixture of step b) to achieve an effective average particle size $D_{50}$ of between 100 - 150 μm when measured with a Beckman Coulter LS13320 Particle size analyzer, wherein in step b) aluminum stearate is added to the solution of step a) before or after adding the levamisole.

According to the invention, the triacetin is the sole liquid carrier in the formulation and in the process no other liquid carrier is introduced.

In a specific process the macrocyclic lactone is eprinomectin, ivermectin or moxidectin.

BHT is also introduced to the solution of step a) and aluminum stearate is introduced to the mixture of macrocyclic lactone solution and levamisole in step b).

In a specific process the levamisole is levamisole HCl.

The method comprises dispersing levamisole particles in a macrocyclic lactone solution in triacetin, adding a dispersion stabilizer and mechanically reducing the particle size of the levamisole particles to an effective average particle size $D_{50}$ of between 100 - 150 μm.

Effective methods of providing mechanical force for particle size reduction of the levamisole to this effective average particle size include colloidal milling, ball milling, media milling, and homogenization, for example with a MICROFLUIDIZER® (Microfluidics Corp.).

Ball milling is a low energy milling process that uses milling media, drug, stabilizer and liquid. The materials are placed in a milling vessel that is rotated at optimal speed such that the media cascades and reduces the drug particle size by impactation. The media used must have a high density as the energy for the particle size reduction is provided by gravity and the mass of the attrition media.

Media milling is a high energy milling process. The formulation of step b) - Drug, stabilizer and liquid are placed in a reservoir and recirculated in a chamber containing media and rotating shaft/impeller. The rotating shaft agitates the media which subjects the drug to impactation and sheer forces, thereby reducing the drug particle size. A media mill is preferred due to the relatively shorter milling time required to provide the intended result, i.e., the desired reduction in particle size. In a specific embodiment a Dyno Mill Type KDL A or Dyno Mill Multi Lab available from WAB, Basel is used.

Alternative mills are Agitated Lab Ball Mill 90 AHM available from Hosokawa Alpine, Augsburg or DCP High Performance Media Mill Megavantis ACS, available from Draiswerke Inc. or DCP Superflow or Advantis perl mills from Bühler.

A colloid mill is used in the disintegration of solid particles or droplet size of a liquid present in suspension or emulsion. The machine consists of an inlet (which is subjected to a tremendous shearing action that effects a time dispersion of uniform size) and an outlet. Colloid mill works on the rotor-stator principle. The equipment breaks down materials by forming dispersion of material in a liquid. Shearing takes place in a narrow gap between a static cone (the stator) and a rapidly rotating cone (the rotor). One example is the IKA colloidal mill, a high-performance inline colloid mill capable of performing wet and fine milling of tough and grainy raw materials. The model IKA MK 2000 is especially suitable for the production of colloidal solutions or extremely fine emulsions and suspensions.

The particles must be reduced in size at a temperature which does not significantly degrade the drug substance, i.e. levamisole or the macrocyclic lactone. If desired, the processing equipment can be cooled with conventional cooling equipment. The method is conveniently carried out under conditions of ambient temperature and at processing pressures which are safe and effective for the milling process. For example, ambient processing pressures are typical of ball mills, attritor mills and vibratory mills. Processing pressures up to about 1.4 $kg/cm^2$ (20 psi) are typical of media milling. In one embodiment the temperature does not exceed 50°C or 40°C.

Particle size reduction by homogenisation as described in U.S. Patent No. 5,510,118 can be used alternatively as a process using a MICROFLUIDIZER® resulting in particles of the desired size.

The current invention provides a process for making the pharmaceutical formulation according to the invention by: dispersing the levamisole particles and the dispersion stabilizer in an ivermectin solution in triacetin; and optionally adding an antioxidant; and mechanically reducing the particle size of the levamisole to an effective average particle size $D_{50}$ of between 100 - 150 $\mu$m.

In the present invention, the particle size $D_{50}$ of the levamisole is reduced by the milling step to between 100 - 150 $\mu$m, or about 110 $\mu$m to about 140 $\mu$m.

**Methods of using the formulation according to the invention to treat parasites**

Formulations within the scope of the invention have been shown to provide efficacy against economically important endo-parasites at up to 4 months, and ecto-parasites at up to 3 months, following a single injection. This represents a significant advantage for those working in the field of livestock animals as it offers effective treatment with one application per season.

In an embodiment the formulations according to this invention are used to treat endoparasites, especially helminth infection, such as an infection caused by one or more helminths selected from the group consisting of *Ancylostoma* spp.; *Anecator* spp.; *Ascaridia* spp.; *Ascaris* spp.; *Brugia* spp.; *Bunostomum* spp.; *Capillaria* spp.; *Chabertia* spp.; *Cooperia* spp.; *Cyathostomum* spp.; *Cylicocyclus* spp.; *Cylicodontophorus* spp.; *Cylicostephanus* spp.; *Craterostomum* spp.; *Dictyocaulus* spp.; *Dipetalonema* spp; *Dirofilaria* spp.; *Dracunculus* spp.; *Enterobius* spp.; *Filaroides* spp.; *Habronema* spp.; *Haemonchus* spp.; *Heterakis* spp.; *Hyostrongylus* spp.; *Metastrongylus* spp.; *Meullerius* spp. *Necator* spp.; *Nematodirus* spp.; *Nippostrongylus* spp.; *Oesophagostomum* spp.; *Onchocerca* spp.; *Ostertagia* spp.; *Oxyuris* spp.; *Parascaris* spp.; *Stephanurus* spp.; *Strongylus* spp.; *Syngamus* spp.; *Toxocara* spp.; *Strongyloides* spp.; *Teladorsagia* spp.; *Toxascaris* spp.; *Trichinella* spp.; *Trichuris* spp.; *Trichostrongylus* spp.; *Triodontophorous* spp.; *Uncinaria* spp., and/or *Wuchereria* spp.

In cattle, the major production limiting parasite species is *Haemonchus spp and Cooperia.* spp. Another important cattle parasite is *Ostertagia.* spp. The formulation of the invention controls *Haemonchus* spp and *Cooperia.* spp when administered to a bovine animal once every 3, 4, 5, 6, 7 or 8 weeks. In another embodiment one administration of the formulation of the invention controls *Haemonchus* spp, *Cooperia* spp and *Ostertagia* spp infestation of animals for 3, 4, 5, 6, 7, 8 or 9 weeks.

In another embodiment one administration of the formulation of the invention controls Ticks (*Rhipicephalus microplus*), Grubs (*larvae of D. hominis*) and Screwworms (larvae of *C. hominivorax*) infestations for 4, 5, 6, 7 or 8 weeks.

The formulations according to this invention may be used to treat animals, especially ruminants such as cattle, sheep and goat. Especially important is the use in bovid animals, such as cattle. In one embodiment the treated animals are dairy cattle, especially during the dry period, or caves or heifers. In another embodiment the treated animals are beef cattle of all age. In one embodiment the beef cattle is in the termination phase (feedlots and pasture), in another embodiment, in cow-calf and growth operations.

In some embodiments, the formulations according to this invention are used to treat an infection by an endoparasite, especially a helminth, such as a nematode, cestode or trematode, preferably a nematode (such as *Haemonchus contortus*), that is resistant to one or more other anthelmintic agents. In some embodiments, the compound according to this invention is active against a helminth, such as a nematode, cestode or trematode, preferably a nematode such as *Haemonchus contortus,* that is resistant to one or more of the following anthelmintics: an avermectin (*e.g.*, ivermectin, selamectin, doramectin, abamectin, and eprinomectin); a milbemycin (moxidectin and milbemycin oxime); a pro-benzimidazole (*e.g.*, febantel, netobimin, and thiophanate); a benzimidazole derivative, such as a thiazole benzimidazole derivative (*e.g.*, thiabendazole and cambendazole) or a carbamate benzimidazole derivative (*e.g.*, fenbendazole, albendazole (oxide), mebendazole, oxfendazole, parbendazole, oxibendazole, flubendazole, and triclabendazole); an imidazothiazole (*e.g.*, levamisole and tetramisole); a tetrahydropyrimidine (morantel and pyrantel), an organophosphate (*e.g.*, trichlorphon, haloxon, dichlorvos, and naphthalophos); a salicylanilide (*e.g.*, closantel, oxyclozanide, rafoxanide, and niclosamide); a nitrophenolic compound (*e.g.*, nitroxynil and nitroscanate); benzoenedisulphonamide (*e.g.*, clorsulon); a pyrazinaisoquinoline (*e.g.*, praziquantel and epsiprantel); a heterocyclic compound (*e.g.*, piperazine, diethylcarbamazine, dichlorophen, and phenothiazine); an arsenical (*e.g.*, thiacetarsamide, melorsamine, and arsenamide); cyclooctadepsipeptide (*e.g.*, emodepside); and a paraherquamide. In another embodiment, the formulations according to this invention are used to treat an infection by an ectoparasites, especially ticks (*R. microplus*), horn flies (*Haematobia irritans*), grubs (*Dermatobia hominis*) and screwworms (*Cochliomyia hominivorax*).

The formulation according to the invention delivers an effective amount of a macrocyclic lactone and levamisole to treat animals against endo and ectoparasites. An example of effective dosage to control helminths such as *Heamonchus* spp, *Cooperia* spp and *Ostertagia* spp and ticks is in the range of about 1- 10 mg/ kg body weight of levamisole and about 0.1 to 1 mg of ivermectin per kg body weight. The injection of an effective amount of the macrocyclic lactione (e.g. ivermectin) and levamisole results in high and early effective blood levels of levamisole and effective blood levels of the macrocyclic lactone (ivermectin) for more than 2 weeks. The formulation according to the invention can be applied to an animal in general by injection administration. Especially preferred is subcutaneous injection. Alternatively the formulation can be administered intramuscularly. In one embodiment the formulation is injected subcutaneously high in the neck or behind the ear of the cattle animal. With the formulation of the current invention no injection site reactions were observed at any time point.

The following examples are provided as examples only and are in no way intended to limit the scope of the invention.

**EXAMPLE FORMULATIONS**

In Table 3, 1 cP = 1 mPas. The formulations F1 and F2 below were prepared as follows. Triacetin was added to a capable vessel with mixing and heated until 55 to 65°C. Ivermectin was added and stirred until dissolved. Butylhydroxyltoluene (BHT) was added. This solution was filtered through a sterilizing membrane to a sterile vessel with agitator under laminar flow. Aluminum stearate and levamisole hydrochloride were mixed with the solution. This mixture was stirred until complete homogenization and passed through a cycling mill to get an homogenous suspension of levamisole. The homogeneous mixture was micronized through a colloid mill while maintaining the temperature below 50°C.

**Formulation (F1)**

Levamisol Hydrochloride - 18.8%(w/v)
Ivermectin - 1%(w/v)
Aluminium Monostearate - 0.5% (w/v)
Butylhydroxytoluene - 0.5% (w/v)
Triacetin qs 100% (v/v)

**Formulation (F2)**

Levamisol Hydrochloride - 18.8%(w/v)
Ivermectin - 4%(w/v)
Aluminium Monostearate - 0.5% (w/v)
Butylhydroxytoluene - 0.5% (w/v)
Triacetin qs 100% (v/v)
Formulations F3 and F4 were prepared as described above for Formulations F1 and F2 except that moxidectin (F3) and eprinomectin (F4) were substituted for ivermectin.

**Formulation (F3)**

Levamisol Hydrochloride - 18.8%(w/v)
Moxidectin - 1%(w/v)
Aluminium Monostearate - 0.5% (w/v)
Butylhydroxytoluene - 0.5% (w/v)
Triacetin qs 100% (v/v)

**Formulation (F4)**

Levamisol Hydrochloride - 18.8%(w/v)

Eprinomectin - 1%(w/v)

Aluminium Monostearate - 0.5% (w/v)

Butylhydroxytoluene - 0.5% (w/v)

Triacetin qs 100% (v/v)

**Comparative Formulation Examples**

Comparative Formulations C1 - C4 were prepared in accordance with the disclosure of International Publication No. WO2011/161209.

Formulation C1

Levamisol Hydrochloride - 18.8%(w/v)

Ivermectin - 2%(w/v)

Dimethylacetamide - 10% (w/v)

Butylhydroxytoluene - 0.02% (w/v)

Castor Oil - 50.4% (w/v)

MCT oil (Crodamol GTCC) qs 100% (v/v)

Formulation C2

Levamisol Hydrochloride - 15.04%(w/v)

Ivermectin - 1.6%(w/v)

Dimethylacetamide - 10% (w/v)

Butylhydroxytoluene - 0.02% (w/v)

Castor Oil - 50.4% (w/v)

MCT oil (Crodamol GTCC) qs 100% (v/v)

Formulation C3

Levamisol Hydrochloride - 18.8%(w/v)

Ivermectin - 4%(w/v)

Dimethylacetamide - 10% (w/v)

Butylhydroxytoluene - 0.02% (w/v)

Castor Oil - 50.4% (w/v)

MCT oil (Crodamol GTCC) qs 100% (v/v)

Formulation C4

Levamisol Hydrochloride - 15.04%(w/v)

Ivermectin - 3.2%(w/v)

Dimethylacetamide - 10% (w/v)

Butylhydroxytoluene - 0.02% (w/v)

Castor Oil - 50.4% (w/v)

MCT oil (Crodamol GTCC) qs 100% (v/v)

Table 2: Additional Comparative Formulations

| Formula S1 | Formula S2 | Formula P1 |
|---|---|---|
| Ivermectin 4.0 % (w/v) | Ivermectin 4.0 % (w/v) | Ivermectin 1.0 % (w/v) |
| Levamisole HCl 15.04-18.8% (w/v) | Levamisole HCl 15.04-18.8% (w/v) | Levamisole HCl 15.04-18.8% (w/v) |
| Dimetylacetamide 10.0% (w/v) | Dimetylacetamide 30.0% (w/v) | Butylated Hydroxytoluene 0.5 % (w/v) |
| Butylated Hydroxytoluene 0.2 % (w/v) | Butylated Hydroxytoluene 0.5 % (w/v) | 2-Pyrrolidone 20.0% (w/v) |

(continued)

| Formula S1 | Formula S2 | Formula P1 |
|---|---|---|
| Sesame Oil 50.4% (w/v) | Sesame Oil qs 100% (v/v) | Glycerol Formal 30.0% (w/v) |
| MCT Oil (Crodamol GTCC) qs 100% (v/v) | | Propylene glycol qs 100% (v/v) |
| SUSPENSION | SUSPENSION | SUSPENSION |

Table 3 Viscosity and Particle Size

| **Test** | **Comparative Formulation C3 from** WO2011/161209 | **Formulation S1** | **Formulation S2** | **Formulation P1** | **New formulations F1-F4** |
|---|---|---|---|---|---|
| Viscosity | 170 to 300cP | 29.8 | 4.17 | 37.2 | 20 to 50cP |
| Density (g/mL) | 0,990 to 1,035 | 0.9863 | 1.071 | 1.1318 | 1,160 to 1,200 |
| Appearance | White to slightly yellowish suspension | White to slightly yellowish suspension | White suspension | White to slightly yellowish suspension | White to slightly yellowish suspension |
| Resuspension | Total Resuspension | Total Resuspension | Total Resuspension | Total Resuspension | Total Resuspension |
| Syringeability | Without clog using a 40 x 1.6 mm needle | Without clog using a 40 x 1.2 mm needle | Without clog using a 40 x 1.2 mm needle | Without clog using a 40 x 1.2 mm needle | Without clog using a 40 x 1.2 mm needle |

Table 4: Stability data (Formulations F1 - F4, C1 - C4, S1 - S2 and P1)

| | INITIAL | | 1st month | | 2nd month | | 3rd month | | 6th month | |
|---|---|---|---|---|---|---|---|---|---|---|
| Batch | Levamisole HCl (%m/v) | Ivermectin(% m/v) | Levamisol e HCl (%m/v) | Ivermectin (% m/v) | Levamisole HCl (%m/v) | Ivermectin (% m/v) | Levamisol e HCl (%m/v) | Ivermectin (% m/v) | Levamisol e HCl (%m/v) | Ivermectin (% m/v) |
| F1 | 19.37 | 0.98 | 19.11 | 1.00 | 18.82 | 0.99 | 19.43 | 0.98 | 19.82 | 0.97 |
| F2 | 20.68 | 3.83 | 20.39 | 4.00 | 20.52 | 4.04 | 21.54 | 4.03 | 19.82 | 3.99 |
| | | | | | | | | | | |
| Batch | Levamisole HCl (%m/v) | Ivermectin (% m/v) | Levamisol e HCl (%m/v) | Ivermectin (%m/v) | Levamisole HCl (%m/v) | Ivermectin (%m/v) | Levamisol e HCl (%m/v) | Ivermectin (%m/v) | Levamisol e HCl (%m/v) | Ivermectin (% m/v) |
| C1 | 18.48 | 1.99 | 18.76 | 2.01 | 19.09 | 1.98 | 19.91 | 2.10 | 18.82 | 2.00 |
| C2 | 15.05 | 1.56 | 14.91 | 1.54 | 15.47 | 1.59 | 15.21 | 1.59 | 15.37 | 1.63 |
| C3 | 14.73 | 3.11 | 14.12 | 3.18 | 14.67 | 3.08 | 14.46 | 3.16 | 14.09 | 3.12 |
| C4 | 19.04 | 3.80 | 18.29 | 3.91 | 18.77 | 3.67 | 18.80 | 4.01 | 18.71 | 3.74 |
| S1 | 17.57 | 3.81 | 17.49 | 3.75 | 16.56 | 3.75 | 16.77 | 3.63 | 16.52 | 3.65 |
| S2 | 18.62 | 3.94 | 18.3 | 3.83 | 17.69 | 3.92 | 17.77 | 3.87 | 17.58 | 3.87 |
| P1 | 19.48 | 1.01 | 19.11 | 0.95 | 19.25 | 0.84 | NA | NA | NA | NA |
| | | | | | | | | | | |
| Batch | Levamisole HCl (%m/v) | Moxidectin (%m/v) | Levamisol e HCl (%m/v) | Moxidectin (%m/v) | Levamisole HCl (%m/v) | Moxidectin (%m/v) | Levamisol e HCl (%m/v) | Moxidectin (%m/v) | Levamisol e HCl (%m/v) | Moxidectin (%m/v) |
| F3 | 19.62 | 1.00 | 19.05 | 1.00 | 19.24 | 1.04 | 19.87 | 0.98 | 19.50 | 0.99 |
| | Levamisole HCl (%m/v) | Eprinomectin (%m/v) | Levamisol e HCl (%m/v) | Eprinomecti n (%m/v) | Levamisole HCl (%m/v) | Eprinomecti n (%m/v) | Levamisol e HCl (%m/v) | Eprinomecti n (%m/v) | Levamisol e HCl (%m/v) | Eprinomectin (%m/v) |
| F4 | 19.93 | 1.09 | 19.07 | 1.07 | 18.97 | 1.08 | 18.74 | 1.10 | 19.40 | |

**Table 5: stability data 50°C ± 2°C / 90% RH ± 5 % of formulation F1**

| 50°C 90%H.R | | | | | | | | |
|---|---|---|---|---|---|---|---|---|
| | INITIAL | | 1st month | | 2nd month | | 3rd month | |
| Batch | Levamisole HCl (%m/v) | Ivermectin (% m/v) | Levamisole HCl (%m/v) | Ivermectin (% m/v) | Levamisole HCl (%m/v) | Ivermectin (% m/v) | Levamisole HCl (%m/v) | Ivermectin (% m/v) |
| F1 | 19.37 | 0.98 | 18.82 | 1.00 | 19.66 | 0.98 | 18.69 | 1.00 |

Formulas F1-F4 proved to be the formulations with both superior stability (see Table 3) and superior syringeability (see Table 4). Each of these formulas survived the accelerated stability test at 40°C± 2°C / 75% RH ± 5% without significant degradation of either of the active ingredients. Moreover, these formulations were able to be syringed without clogging using the smaller 40x1.2 mm syringe. Formulation C3 had acceptable stability but could only be syringed without clogging using a larger needle (40x1.6 mm). Formulations S1, S2 and P1 were syringed acceptably by the smaller needle but were unstable for their active ingredients which degraded more than 5% during the 6 month accelerated stability study.

**Anthelmintic efficacy of two experimental formulations containing 4% Ivermectin + 18.8% Levamisole administered by subcutaneous route for the treatment of cattle naturally infected with gastrointestinal nematodes**

Experimental Procedures:

18 crossbreed steers, with age between 7 and 12 months, were naturally infected with gastrointestinal nematodes. Animals were randomly distributed in 3 groups, based on pre-treatment nematode EPG (eggs per gram of feces) counts.

| Group | # of animals | Treatment | Route* | Dose |
|---|---|---|---|---|
| I | 6 | Saline Control | | 1 mL/50kg |
| II | 6 | GLA Oil-based | SC | 1 mL/50kg |
| III | 6 | GLA Aqueous | | 1 mL/50kg |
| * SC = subcutaneous | | | | |

Formulations

1. **GLA Oil-Base:**

| Raw Material | w/V - 100mL |
|---|---|
| Ivermectin | 4,0 g |
| Levamisole HCl | 18,8 g |
| Aluminum Stearate | 0,5 g |
| Butylated Hydroxytoluene | 0,5 g |
| Triacetin | qs. |

2. **GLA Aqueous:** not according to the invention

| Raw Material | w/V - 100mL |
|---|---|
| Ivermectin | 4,0 g |
| Levamisole HCl | 18,8 g |
| Aluminum Hydroxide | 20,0 g |
| Povidone | 10,0 g |
| Antifoam agent (Simethicone) | 0,2 g |
| Surfactant agent (Tween 80) | 0,35 g |
| Sodium Metabisulfite | 0,2 g |
| Methylparaben | 0,18 g |

(continued)

| Raw Material | w/V - 100mL |
|---|---|
| Propylparaben | 0,02 g |
| WFI Water | qs |

**Treatments**

On D0, the animals of groups I, II and III were treated with the respective formulations. The administration was done by subcutaneous route on the lateral midline of neck using suitable aseptic techniques.

**EPG Counts/ Larvae Cultures**

Faeces were collected directly from the animal's rectum. EPG counts were carried out as described by GORDON & WHITLOCK on days D-3, D-2 and D-1 (pre-treatment) and D+2, D+4, D+6, D+8, D+10, D+12 and D+14 (post-treatment). Larvae cultures were performed on the same EPG counting days (until D+12).

**Necropsy**

On days 14th and 15th post treatment, animals were slaughtered. In each of the study days, 9 animals (3 from each group) were sacrificed. Parasite collection, counting and genus identification was carried out using stereoscopic microscope (magnifier).

**Efficacy**

Arithmetic means were used to estimate percent reduction of helminthes for each group, using MAPA's recommended formula (Brazil, Ordinance 48, 1997):

$$\text{Efficacy\%} = \frac{\text{Mean number of Helminths CG} - \text{Mean number of Helminths TG}}{\text{Mean number of Helminths CG}} \times 100$$

Where:

CG = Control Group;
TG = Treated Group

**Results**

**a) Pre-slaughtering EPG Counts and Efficacy**

**Table 5.** Pre-slaughtering EPG Counts and Efficacy

| Group | Animal ID | EPG Count | | | | | | | |
|---|---|---|---|---|---|---|---|---|---|
| | | Mean (-3, -2, - 1) | +2 | +4 | +6 | +8 | +10 | +12 | +14 |
| Saline Control | 1 | 183 | 200 | 175 | 375 | 125 | 175 | 50 | 150 |
| | 133 | 308 | 350 | 325 | 325 | 675 | 250 | 200 | 775 |
| | 137 | 133 | 25 | 25 | 50 | 25 | 100 | 50 | 50 |
| | 146 | 225 | 375 | 125 | 250 | 400 | 275 | 275 | 425 |
| | 148 | 917 | 1150 | 1200 | 1050 | 900 | 1125 | 1225 | 1100 |
| | 149 | 917 | 1200 | 675 | 625 | 1150 | 1800 | 1600 | 900 |
| **Mean** | | **447** | **550** | **421** | **446** | **546** | **621** | **567** | **567** |

(continued)

| Group | Animal ID | EPG Count | | | | | | | |
|---|---|---|---|---|---|---|---|---|---|
| | | Mean (-3, -2, - 1) | +2 | +4 | +6 | +8 | +10 | +12 | +14 |
| GLA Oil-based | 24 | 525 | 0 | 0 | 0 | 125 | 0 | 0 | 0 |
| | 109 | 525 | 0 | 0 | 0 | 0 | 25 | 0 | 0 |
| | 115 | 192 | 0 | 0 | 0 | 0 | 0 | 0 | 0 |
| | 123 | 100 | 0 | 0 | 0 | 0 | 0 | 0 | 0 |
| | 128 | 1242 | 0 | 0 | 0 | 0 | 0 | 0 | 0 |
| | 135 | 117 | 0 | 0 | 0 | 0 | 0 | 0 | 0 |
| **Mean** | | **450** | **0** | **0** | **0** | **21** | **4** | **0** | **0** |
| **Efficacy (%)** | | | **100.0** | **100.0** | **100.0** | **96.2** | **99.3** | **100.0** | **100.0** |
| GLA Aqueous | 2 | 117 | 0 | 0 | 0 | 0 | 0 | 0 | 25 |
| | 112 | 292 | 0 | 0 | 0 | 0 | 0 | 0 | 0 |
| | 118 | 258 | 0 | 0 | 0 | 0 | 0 | 25 | 0 |
| | 127 | 725 | 125 | 25 | 25 | 0 | 25 | 0 | 50 |
| | 141 | 1158 | 0 | 0 | 0 | 0 | 0 | 0 | 50 |
| | 147 | 117 | 0 | 0 | 0 | 0 | 0 | 0 | 0 |
| **Mean** | | **445** | **21** | **4** | **4** | **0** | **4** | **4** | **21** |
| **Efficacy (%)** | | | **96.2** | **99.0** | **99.1** | **100.0** | **99.3** | **99.3** | **96.3** |

b) **Pre-slaughtering Larvae Cultures**

**Table 6:** Pre-slaughtering Larvae Cultures

| Exp. Day | Treatmen t | Helminthes genus (%) | | | |
|---|---|---|---|---|---|
| | | *Cooperi a* spp | *Haemonchu s* spp | *Oesophagostomu m* spp | *Trichostrongylu s* spp |
| **-3** | N/A (pool) | 23.4 | 71 | 5.6 | 0 |
| **-2** | | 37 | 54.6 | 8.4 | 0 |
| **-1** | | 29.3 | 61 | 9.3 | 0.4 |
| **Mean** | | **29,9** | **62.2** | **7.8** | **0.1** |
| **+2** | Control | 24.7 | 66 | 7.3 | 2 |
| | Oil-based | ND | ND | ND | ND |
| | Aqueous | 19 | 80.5 | 0.5 | 0 |
| **+4** | Control | 9.3 | 81 | 2.7 | 7 |
| | Oil-based | ND | ND | ND | ND |
| | Aqueous | 14 | 86 | 0 | 0 |
| **+6** | Control | 53.6 | 36.2 | 10.1 | 0 |
| | Oil-based | ND | ND | ND | ND |
| | Aqueous | ND | ND | ND | ND |
| **+8** | Control | 14.3 | 79.3 | 6.4 | 0 |
| | Oil-based | ND | ND | ND | ND |
| | Aqueous | ND | ND | ND | ND |
| **+10** | Control | 27 | 53 | 20 | 0 |
| | Oil-based | ND | ND | ND | ND |
| | Aqueous | ND | ND | ND | ND |

(continued)

| Exp. Day | Treatmen t | Helminthes genus (%) | | | |
|---|---|---|---|---|---|
| | | ***Cooperi a*** **spp** | ***Haemonchu s*** **spp** | ***Oesophagostomu m*** **spp** | ***Trichostrongylu s*** **spp** |
| **+12** | Control | 50 | 45 | 5 | 0 |
| | Oil-based | ND | ND | ND | ND |
| | Aqueous | ND | ND | ND | ND |

* ND = not detected

c) **Post-slaughtering/ necropsy efficacy (Helminthes Genus)**

| | | Genus | | | | | | | | |
|---|---|---|---|---|---|---|---|---|---|---|
| Control | | ***Haem.* sp. Adult** | ***Haem.* sp. Imature** | **Coop.sp. Adult** | **Coop.sp. Imature** | **Oesoph.sp. Adult** | **Oesoph.sp. Imature** | ***Trichost.* sp.** | ***Trichuris* sp.** | **Total** |
| | Mean | 438.2 | 23.4 | 870.5 | 35.5 | 24.0 | 14.0 | 0.8 | 0.8 | 1028 |
| | Std. Dev | 183.5 | 26.9 | 956.4 | 51.5 | 28.2 | 20.2 | 2.0 | 1.3 | 984 |
| GLA Oil-based | | | | | | | | | | |
| | Mean | 5.0 | 0.2 | 10.0 | 0.8 | 0.2 | 0.2 | 0.0 | 0.0 | 16 |
| | Std. Dev | 9.4 | 0.4 | 12.2 | 1.3 | 0.4 | 0.4 | 0.0 | 0.0 | 18 |
| | Efficacy (%) | **98.9** | **99.3** | **98.9** | **97.7** | **99.3** | **98.8** | **100.0** | **100.0** | **98.4** |
| Aqueous | | | | | | | | | | |
| | Mean | 39.7 | 1.8 | 12.8 | 1.8 | 2.2 | 2.2 | 0.0 | 1.2 | 62 |
| | Std. Dev | 59.6 | 2.7 | 2.6 | 2.2 | 5.3 | 5.3 | 0.0 | 2.0 | 68 |
| | Efficacy (%) | **90.9** | **92.2** | **98.5** | **94.8** | **91.0** | **84.5** | **100.0** | **0.0** | **94.0** |

**Conclusions**

- Pre-slaughtering EPG counts were better for the GLA oil-based formula.
- Post-slaughtering/ necropsy showed better results for the oil-based formula as the total efficacy was 98.4% (considered highly effective by the Brazilian authorities) compared to 94% total efficacy of the aqueous formula.
- For all helminthes genus, the efficacy of the oil-based formula was >95%, which is the minimum required efficacy by the Brazilian authorities.
- The aqueous based formula only achieved efficacy > 95% for the *Cooperia* spp (Adult) and *Trichostrongylus* spp genus.

## Claims

1. An injectable pharmaceutical formulation comprising a macrocyclic lactone selected from the group comprising abamectin, doramectin, eprinomectin, ivermectin and moxidectin and levamisole, and triacetin as the sole liquid carrier and a dispersion stabilizer selected from metallic stearates of calcium, zinc, magnesium or aluminum, wherein the macrocyclic lactone is in solution and the levamisole is in suspension and has an average effective particle size $D_{50}$ between 100 - 150 μm when measured with a Beckman Coulter LS13320 Particle size analyzer.

2. The formulation of claim 1, wherein the dispersion stabilizer is aluminum stearate.

3. The formulation of claim 1, wherein the formulation comprises no surfactant other than metallic stearates of calcium, zinc, magnesium or aluminium.

4. The formulation of claim 1, 2 or 3, wherein the formulation comprises 0.1 to 1 % w/v of the dispersion stabilizer.

5. The formulation of any one of claims 1 to 4, wherein the formulation further comprises a preservative, preferably butylated hydroxytoluene.

6. The formulation of any one of claims 1 to 5, wherein the suspended levamisole is present in a range of between 15-20% w/v.

7. The formulation of any one of claims 1 to 6, wherein the macrocyclic lactone compound is present in a range of between 0.5-10% w/v.

8. The formulation of any one of claims 1 to 7, wherein the macrocyclic lactone compound is selected from the group comprising eprinomectin, ivermectin and moxidectin.

9. The formulation of any one of claims 1 to 8, wherein the levamisole is the hydrochloride salt.

10. The formulation of any one of claims 1 to 9, wherein the levamisole is present in 18% w/v.

11. The formulation of any one of claims 1 to 10, wherein the macrocyclic lactone is ivermectin and is present in 1 or 4% w/v.

12. The formulation of claim 1, comprising:

    - 0.5- 10% w/v ivermectin,
    - 15- 20% w/v levamisole,
    - 0.1-1% w/v aluminium monostearate,
    - 0.1-1% w/v butylated hydroxytoluene and
    - triacetin, qs 100% (v/v).

**13.** The formulation of any one of the claims 1 to 12 for use in treating or preventing infestation or infection of internal parasites and external parasites in animals.

**14.** A process for preparing an injectable pharmaceutical formulation according to any one of the claims 1 to 12 comprising

a) dissolving a macrocyclic lactone in triacetin;
b) mixing the macrocyclic lactone solution with levamisole; and
c) milling the mixture of step b) to achieve an effective average particle size $D_{50}$ of between 100 - 150 μm when measured with a Beckman Coulter LS13320 Particle size analyzer,

wherein in step b) aluminum stearate is added to the solution of step a) before or after adding the levamisole.

**15.** The process according to claim 14, wherein the temperature of step c) is kept below 50°C.

**Patentansprüche**

**1.** Injizierbare pharmazeutische Zusammensetzung, umfassend ein makrocyclisches Lacton aus der Gruppe umfassend Abamectin, Doramectin, Eprinomectin, Ivermectin und Moxidectin und Levamisol und Triazin als einzigen flüssigen Träger und einen Dispersionsstabilisator, der aus Metallstearaten von Calcium, Zink, Magnesium oder Aluminium ausgewählt ist, wobei das makrocyclische Lacton in Lösung vorliegt und das Levamisol in Suspension vorliegt und eine mittlere effektive Teilchengröße $D_{50}$ zwischen 100-150 μm bei Messung mit einem Teilchengrößenanalysator des Typs Beckman Coulter LS 13320 aufweist.

**2.** Formulierung nach Anspruch 1, wobei es sich bei dem Dispersionsstabilisator um Aluminiumstearat handelt.

**3.** Formulierung nach Anspruch 1, wobei die Formulierung kein anderes Tensid als Metallstearate von Calcium, Zink, Magnesium oder Aluminium umfasst.

**4.** Formulierung nach Anspruch 1, 2 oder 3, wobei die Formulierung 0,1 bis 1 % w/v des Dispersionsstabilisators umfasst.

**5.** Formulierung nach einem der Ansprüche 1 bis 4, wobei die Formulierung ferner einen Konservierungsstoff, vorzugsweise Butylhydroxytoluol, umfasst.

**6.** Formulierung nach einem der Ansprüche 1 bis 5, wobei das suspendierte Levamisol in einem Bereich zwischen 15-20 % w/v vorliegt.

**7.** Formulierung nach einem der Ansprüche 1 bis 6, wobei die makrocyclische Lactonverbindung in einem Bereich zwischen 0,5-10 % w/v vorliegt.

**8.** Formulierung nach einem der Ansprüche 1 bis 7, wobei die makrocyclische Lactonverbindung aus der Gruppe umfassend Eprinomectin, Ivermectin und Moxidectin ausgewählt ist.

**9.** Formulierung nach einem der Ansprüche 1 bis 8, wobei es sich bei dem Levamisol um das Hydrochloridsalz handelt.

**10.** Formulierung nach einem der Ansprüche 1 bis 9, wobei das Levamisol in einer Menge von 18 % w/v vorliegt.

**11.** Formulierung nach einem der Ansprüche 1 bis 10, wobei das makrocyclische Lacton Ivermectin ist und in einer Menge von 1 oder 4 % w/v vorliegt.

**12.** Formulierung nach Anspruch 1, umfassend:

- 0,5-10 % w/v Ivermectin,
- 15-20 % w/v Levamisol,
- 0,1-1 % w/v Aluminiummonostearat,
- 0,1-1 % w/v Butylhydroxytoluol und
- Triacetin q.s. 100 % (v/v).

**13.** Formulierung nach einem der Ansprüche 1 bis 12 zur Verwendung bei der Behandlung oder Prävention von Befall oder Infektion durch interne Parasiten und externe Parasiten bei Tieren.

**14.** Verfahren zur Herstellung einer injizierbaren pharmazeutischen Formulierung nach einem der Ansprüche 1 bis 12, das Folgendes umfasst:

a) Lösen eines makrocyclischen Lactons in Triacetin;
b) Mischen der Lösung des makrocyclischen Lactons mit Levamisol und
c) Mahlen der Mischung aus Schritt b) zur Erzielung einer effektiven Teilchengröße $D_{50}$ zwischen 100-150 $\mu$m bei Messung mit einem Teilchengrößenanalysator des Typs Beckman Coulter LS 13320,

wobei in Schritt b) Aluminiumstearat vor oder nach der Zugabe des Levamisols zu der Lösung aus Schritt a) gegeben wird.

**15.** Verfahren nach Anspruch 14, wobei die Temperatur von Schritt c) unter 50 °C gehalten wird.

**Revendications**

**1.** Formulation pharmaceutique injectable comprenant une lactone macrocyclique choisie dans le groupe comprenant l'abamectine, la doramectine, l'éprinomectine, l'ivermectine et la moxidectine et le lévamisole, et de la triacétine en tant qu'unique support solide et un stabilisateur de dispersion choisi parmi des stéarates métalliques de calcium, de zinc, de magnésium et d'aluminium, la lactone macrocyclique étant en solution et le lévamisole étant en suspension et possédant une taille de particule effective moyenne $D_{50}$ comprise entre 100 et 150 $\mu$m lorsqu'elle est mesurée avec un analyseur de taille de particule Beckman Coulter LS13320.

**2.** Formulation selon la revendication 1, le stabilisateur de dispersion étant le stéarate d'aluminium.

**3.** Formulation selon la revendication 1, la formulation ne comprenant pas de tensioactif autre que des stéarates métalliques de calcium, de zinc, de magnésium ou d'aluminium.

**4.** Formulation selon la revendication 1, 2 ou 3, la formulation comprenant 0,1 à 1 % p/v du stabilisateur de dispersion.

**5.** Formulation selon l'une quelconque des revendications 1 à 4, la formulation comprenant en outre un conservateur, préférablement de l'hydroxytoluène butylé.

**6.** Formulation selon l'une quelconque des revendications 1 à 5, le lévamisole en suspension étant présent dans une plage comprise entre 15 et 20 % p/v.

**7.** Formulation selon l'une quelconque des revendications 1 à 6, le composé de type lactone macrocyclique étant présent dans une plage comprise entre 0,5 et 10 % p/v.

**8.** Formulation selon l'une quelconque des revendications 1 à 7, le composé de type lactone macrocyclique étant choisi dans le groupe comprenant l'éprinomectine, l'ivermectine et la moxidectine.

**9.** Formulation selon l'une quelconque des revendications 1 à 8, le lévamisole étant le sel de chlorhydrate.

**10.** Formulation selon l'une quelconque des revendications 1 à 9, le lévamisole étant présent en une quantité de 18 % p/v.

**11.** Formulation selon l'une quelconque des revendications 1 à 10, la lactone macrocyclique étant l'ivermectine et étant présente en une quantité de 1 ou 4 % p/v.

**12.** Formulation selon la revendication 1, comprenant :

- 0,5 à 10 % p/v d'ivermectine,
- 15 à 20 % p/v de lévamisole,
- 0,1 à 1 % p/v de monostéarate d'aluminium,
- 0,1 à 1 % p/v d'hydroxytoluène butylé et

• de la triacétine, qsp 100 % (v/v).

**13.** Formulation selon l'une quelconque des revendications 1 à 12 pour une utilisation dans le traitement ou la prévention d'une infestation ou d'une affection par des parasites internes et des parasites externes chez des animaux.

**14.** Procédé pour la préparation d'une formulation pharmaceutique injectable selon l'une quelconque des revendications 1 à 12 comprenant

a) la dissolution d'une lactone macrocyclique dans de la triacétine ;
b) le mélange de la solution de lactone macrocyclique avec du lévamisole ; et
c) le broyage du mélange de l'étape b) pour atteindre une taille de particule moyenne effective $D_{50}$ comprise entre 100 et 150 $\mu$m lorsqu'elle est mesurée avec un analyseur de taille de particule Beckman Coulter LS13320,

dans lequel dans l'étape b), du stéarate d'aluminium est ajouté à la solution de l'étape a) avant ou après l'ajout du lévamisole.

**15.** Procédé selon la revendication 14, la température de l'étape c) étant maintenue en dessous de 50 °C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0074489 A
- NZ 280085280134
- WO 00061068 A
- WO 2004009080 A
- BR P19802431
- WO 2011161209 A
- CN 1375291
- EP 0413538 A
- NZ 594610
- WO 2013030702 A2
- WO 2008072985 A
- US 4199569 A
- US 4310519 A
- US 5089480 A
- US 5288710 A
- US 5399717 A
- US 3950360 A
- US 3984564 A
- US 4916154 A
- US 5510118 A


### Non-patent literature cited in the description


- The Merck Veterinary Manual. 1998, 1812-1814
- The Condensed Chemical Dictionary. 1956, 1159
- **GENNARO.** Remington: The Science and Practice of Pharmacy. 2000
- **BRAZIL.** *Ordinance,* 1997, vol. 48